# FASCICULE DE BREVET EUROPEEN

(11) **EP 0 605 595 B1**
(45) Date de publication et mention de la délivrance du brevet: **06.12.1995**
(21) Numéro de dépôt: 92920775.1
(22) Date de dépôt: 16.09.1992
(51) Int. Cl.: A61K 7/00, A61K 7/13, C09B 67/00

(54) **DISPERSION FINE DE PIGMENTS MELANIQUES, SA PREPARATION ET SON UTILISATION EN COSMETIQUE**
FEINE DISPERSION VON MELANINPIGMENTE, SEINE HERSTELLUNG SOWIE SEINE VERWENDUNG IN DIE KOSMETIK
FINE DISPERSION OF MELANIC PIGMENTS, ITS PREPARATION AND UTILISATION IN COSMETICS

(30) Priorité: 18.09.1991 FR 9111514
(43) Date de publication de la demande: 13.07.1994
(73) Titulaire: L'OREAL, 75008 Paris (FR)
(72) Inventeur: ANDREAN, Hervé, F-75014 Paris (FR); CANDAU, Didier, F-91570 BIEVRES (FR); MELLUL, Myriam, F-94240 L'Hay-les-Roses (FR); PAPANTONIOU, Christos, F-95160 Montmorency (FR); PIOT, Bertrand, F-92250 La Garenne-Colombes (FR)
(74) Mandataire: Casalonga, Axel
(86) Numéro de dépôt international: FR9200868
(87) Numéro de publication internationale: WO9305754

(56) Documents cités:
- EP-A- 0 042 816
- EP-A- 0 313 380
- EP-A- 0 386 680
- EP-A- 0 441 689
- EP-A- 0 467 767
- WO-A-90/01919
- WO-A-92/05761
- GB-A- 2 207 153

## Description

La présente invention a pour objet une dispersion fine de pigments mélaniques, son procédé de préparation et son utilisation en cosmétique.

Les pigments mélaniques sont des pigments connus en eux-mêmes. Il s'agit plus particulièrement des pigments qui sont à l'origine de la coloration des cheveux, de la peau ou des poils d'origine humaine ou animale. Ils peuvent également être préparés par synthèse, en particulier par oxydation de dérivés indoliques tels que plus particulièrement le 5,6-dihydroxyindole.

Les pigments mélaniques ayant une faible granulométrie sont particulièrement intéressants dans la mesure où ils présentent un bon pouvoir couvrant, ce qui permet de les utiliser dans des concentrations moindres comparativement aux pigments ayant une granulométrie plus élevée en vue d'obtenir la même coloration.

L'intérêt des pigments ayant une faible granulométrie réside également dans leurs caractéristiques cosmétiques, notamment en ce qui concerne le toucher. Les compositions à base de pigments de faible granulométrie ont en effet un toucher plus doux.

Les pigments mélaniques connus ont une granulométrie généralement comprise entre 100 et 150 »m et doivent être broyés par exemple au mortier, par un broyeur, par micronisation ou par d'autres techniques de broyage, afin d'obtenir des particules ayant une granulométrie comprise entre 15 et 25 »m.

Pour obtenir une granulométrie plus faible, il est encore nécessaire de broyer ces pigments, ce qui s'effectue généralement en milieu aqueux. On constate cependant à la suite de ce broyage et même après séchage du pigment, une réagglomération des différentes particules.

Les compositions cosmétiques contenant des pigments présentés de cette façon ont souvent des caractéristiques peu cosmétiques, dans la mesure où les particules sont relativement grosses et irrégulières. Du fait de l'agglomération, les compositions sont inesthétiques, souvent peu stables et peu couvrantes.

La demande WO 92/05761 est un droit antérieur non publié et décrit des compositions contenant une dispersion fine de vésicules lipidiques contenant un pigment mélanique dans la phase aqueuse externe.

La demande européenne EP 0467.767 qui est un droit antérieur non publié a pour objet des produits à base de particules lamellaires comportant un pigment mélanique.

Le brevet GB A 2.207153 décrit une composition cosmétique constituée par des particules inorganiques ultrafines revêtues par un pigment mélanique.

La demanderesse a constaté qu'en effectuant le broyage du pigment mélanique dans un milieu particulier, il était possible d'obtenir une dispersion fine d'un pigment mélanique, stable dans le temps, ne se réagglomérant pas. La dispersion ainsi obtenue présente par ailleurs l'avantage de pouvoir être utilisée telle quelle dans des compositions cosmétiques sans qu'il soit nécessaire d'isoler, de sécher et de remettre en suspension le pigment.

Ces dispersions présentent par ailleurs des propriétés antioxydantes améliorées.

L'invention a donc pour objet une dispersion stable de pigments mélaniques, ayant une granulométrie très fine, de faible dispersité dans un milieu tel que défini ci-après.

Un autre objet de l'invention est constitué par le procédé de préparation d'une telle dispersion.

L'invention a également pour objet l'utilisation de cette dispersion dans la préparation de compositions cosmétiques et aux compositions cosmétiques mettant en oeuvre cette dispersion.

D'autres objets de l'invention apparaîtront à la lecture de la description et des exemples qui suivent.

Le procédé de préparation des dispersions de pigments mélaniques conforme à l'invention, consiste à broyer un pigment mélanique dans un milieu liquide constitué par :
a) un liquide choisi parmi :
   - un mono-alcool linéaire ou ramifié comportant au moins 3 atomes de carbone;
   - un polyol linéaire ou ramifié comportant au moins 2 atomes de carbone;
   - un alcool cyclo-aliphatique saturé ou insaturé comportant au moins 6 atomes de carbone;
   - un alcool aromatique comportant au moins 6 atomes de carbone;
   - un ester d'acide gras en C₈-C₂₂ et d'alcool en C₁-C₆ ou de polyol en C₂-C₆, un ester d'acide aliphatique en C₁-C₆ ou d'acide aromatique et d'alcool gras en C₈-C₂₂, un ester d'acide gras en C₈-C₂₂ et d'alcool gras en C₈-C₂₂, un ester de polyacide et d'alcool en C₆-C₂₂, un diester d'acide gras hydroxylé;
   - une paraffine linéaire ou ramifiée comportant au moins 8 atomes de carbone;
   - une huile de silicone;
   - ou un mélange d'un ou plusieurs des composés définis ci-dessus,
   ou
b) une émulsion constituée par un liquide défini ci-dessus non miscible à l'eau, de l'eau et un agent émulsionnant.

Le pigment mélanique est présent dans la phase liquide utilisée lors de l'étape de broyage à une concentration généralement comprise entre 5 et 50% et de préférence entre 15 et 35%.

Le procédé est mis en oeuvre à une température comprise entre la température ambiante et 110°C. Les moyens de broyage sont constitués de préférence par un broyeur à billes. On utilise plus particulièrement des billes d'oxyde de zirconium, des billes de verre ou du sable.

La durée du broyage dépend de la nature du pigment, de la grosseur des particules de pigments mélaniques, de la nature du milieu liquide, et des paramètres de broyage. La durée est généralement comprise entre 15 minutes et 24 heures et plus préférentiellement entre 30 minutes et 9 heures.

On utilise de préférence un broyeur à billes vendu sous la dénomination DYNOMILL, type KDL Spécial, dont les billes sont en oxyde de zirconium, ayant un diamètre compris entre 1 et 1,25 mm.

Selon une mise en oeuvre préférée du procédé, on effectue la préparation des dispersions de pigments mélaniques conformes à l'invention, en dispersant dans un premier temps des pigments mélaniques ayant de préférence une granulométrie comprise entre 15 et 20 »m dans le milieu liquide de broyage tel que défini ci-dessus en présence de billes telles que plus particulièrement des billes d'oxyde de zirconium et on procède au broyage jusqu'à ce qu'au moins 75% des particules aient une granulométrie inférieure à 10 »m et de préférence au moins 85% des particules aient une granulométrie inférieure à 5 »m.

Selon un mode préféré, on disperse environ 1 partie de pigment mélanique ayant une granulométrie de 15 à 25 »m dans environ 2 parties de milieu liquide défini ci-dessus. L'on procède au broyage en présence d'environ 3 parties de billes.

Le milieu de broyage défini est liquide. Si on utilise des composés solides à température ambiante, il sera nécessaire de chauffer le milieu à une température légèrement supérieure au point de fusion de ces composés avant de disperser le pigment mélanique dans ce milieu et d'effectuer le broyage.

Le pigment mélanique utilisé conformément à l'invention peut être d'origine naturelle ou synthétique. Les pigments synthétiques sont en particulier des pigments résultant de la polymérisation oxydante d'un composé indolique répondant à la formule : 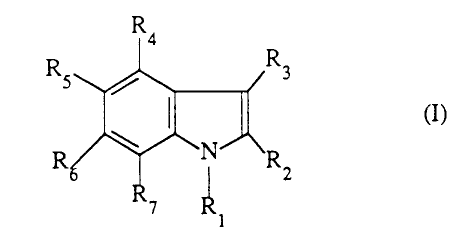
dans laquelle :
R₁ et R₃ représentent, indépendamment l'un de l'autre, un atome d'hydrogène ou un groupe alkyle en C₁-C₄;
R₂ représente un atome d'hydrogène, un groupe alkyle en C₁-C₄, un groupe carboxyle ou un groupe alcoxy(C₁-C₄)carbonyle;
R₄ et R₇ représentent, indépendamment l'un de l'autre, un atome d'hydrogène, un groupe hydroxy, un groupe alkyle en C₁-C₄, un groupe amino, un groupe alcoxy en C₁-C₄, un groupe acyl(C₂-C₄)oxy ou un groupe acyl(C₂-C₄)amino;
R₅ représente un atome d'hydrogène, un groupe hydroxy, un groupe alcoxy en C₁-C₄, un groupe alkyle en C₁-C₄, un atome d'halogène, un groupe amino, un groupe acyl(C₂-C₁₄)oxy, un groupe acyl(C₂-C₄)amino ou un groupe triméthylsilyloxy;
R₆ représente un atome d'hydrogène, un groupe hydroxy, un groupe alcoxy en C₁-C₄, un groupe amino, un groupe acyl(C₂-C₄)oxy, un groupe acyl(C₂-C₄)amino, un groupe triméthylsilyloxy ou un groupe hydroxyalkyl(C₂-C₄)amino;
R₅ et R₆ pouvant également former, conjointement avec les atomes de carbone auxquels ils sont rattachés, un cycle carbonyldioxy;
au moins l'un des radicaux R₄ à R₇ représente un groupement OZ ou NHR₈, l'un au plus des radicaux R₄ à R₇ représentant NHR₈ et deux au plus des radicaux R₄ à R₇ représentant OZ et, dans le cas où Z représente un atome d'hydrogène, les deux groupes OH sont dans les positions 5 et 6; et au moins l'un des radicaux R₄ à R₇ représente un atome d'hydrogène, et dans le cas où un seul de ces radicaux R₄ à R₇ représente un atome d'hydrogène, alors un seul radical parmi R₄ à R₇ représente NHR₈ ou OZ, les autres radicaux représentant un groupe alkyle en C₁-C₄;
le radical R₈ du groupement NHR₈ désignant un atome d'hydrogène, un groupe acyle en C₂-C₄ ou hydroxyalkyle en C₂-C₄, et le radical Z du groupement OZ désignant un atome d'hydrogène, un groupe acyle en C₂-C₁₄, un groupe alkyle en C₁-C₄, ou un groupe triméthylsilyle,
et leurs sels de métaux alcalins, alcalino-terreux, d'ammonium ou d'amines.

Les composés indoliques de formule (I) ci-dessus, sont choisis parmi le 4-hydroxyindole, le 5-hydroxyindole, le 6-hydroxyindole, le 7-hydroxyindole, le 4-hydroxy 5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthylindole, le 2-carboxy 6-hydroxyindole le 6-hydroxy N-méthyl indole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N,β-hydroxyéthyl aminoindole, le 4-aminoindole, le 5-aminoindole, le 6-aminoindole, le 7-aminoindole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-triméthylsilyloxyindole, l'ester phosphorique du 5,6-dihydroxyindole, le 5,6-dibenzyloxyindole, et les sels d'addition de ces composés.

Les composés indoliques particulièrement préférés sont : le 5,6-dihydroxyindole, le 6-hydroxyindole, le 7-hydroxyindole, le bromhydrate de 2-méthyl 5,6-dihydroxyindole, le 7-aminoindole, le 3-méthyl 5,6-dihydroxyindole, le 4-hydroxy 5-méthoxyindole, le 2,3-diméthyl 5-méthoxy 6-hydroxyindole.

La polymérisation oxydante des composés de formule (I) peut s'effectuer en milieu aqueux, eau/solvant(s), ou solvant(s) à l'air, en présence ou non d'un agent alcalin et/ou d'un agent oxydant tel que le peroxyde d'hydrogène, de préférence en présence d'un agent alcalin tel que l'ammoniaque ou en présence d'ions iodure, l'iodure étant de préférence un iodure de métal alcalin, alcalino-terreux ou d'ammonium.

L'oxydation du composé de formule (I) peut également être effectuée en utilisant l'acide periodique et ses sels hydrosolubles et dérivés, les permanganates et bichromates, tels que de sodium ou de potassium, l'hypochlorite de sodium, le persulfate d'ammonium, le nitrite de sodium et des oxydants organiques choisis parmi les ortho- et parabenzoquinones, les ortho- et parabenzoquinones mono- ou diimines, les 1,2- et 1,4-naphtoquinones, les 1,2- et 1,4-naphtoquinones mono- ou diimines telles que décrites dans la demande EP-A-0376 776. Le sel d'acide periodique préféré est le periodate de sodium.

Il est possible d'activer les agents oxydants par un modificateur de pH.

Le procédé de polymérisation oxydante préféré met en oeuvre le peroxyde d'hydrogène en présence d'ammoniaque. Cette réaction d'oxydation s'effectue généralement à une température de l'ordre de 20°C à 100°C, et de préférence 60°C à 90°C.

La réalisation de la polymérisation oxydante s effectue de préférence en introduisant le composé indolique de formule (I) dans un milieu aqueux, ou dans un mélange d'eau et d'un ou plusieurs solvants pouvant contenir jusqu'à 95% de solvant ou encore dans un ou plusieurs solvants anhydre(s), c'est-à-dire contenant moins de 1% d'eau.

Parmi les solvants utilisables, on peut citer les alcools inférieurs en C₁-C₄ tels que l'alcool éthylique, l'alcool propylique ou isopropylique, l'alcool tert.-butylique, les alkylèneglycols tels que l'éthylèneglycol, le propylèneglycol, les alkyléthers d'alkylèneglycols tels que les éthers monométhylique, monoéthylique ou monobutylique de l'éthylèneglycol, les monométhyléthers du propylèneglycol et du dipropylèneglycol, et des esters tels que le lactate de méthyle. Le milieu solvant préféré est un milieu hydroalcoolique contenant de 1 à 10% d'alcool éthylique.

Suivant les procédés, on laisse l'oxydant et le composé indolique de formule (I) en contact pendant quelques minutes à quelques jours.

Les agents alcalinisants sont de préférence choisis parmi l'hydroxyde de sodium, les carbonates alcalins ou l'ammoniaque, dans des proportions comprises entre 5 x 10⁻⁴ % à 10% en poids par rapport au poids de la composition soumise à l'oxydation.

Lorsqu'on utilise un iodure en présence de peroxyde d'hydrogène, on utilise de préférence l'iodure de sodium ou de potassium à une concentration comprise entre 1 et 6%.

Le pigment coloré résultant de la polymérisation oxydante est obtenu sous forme insoluble. Il est isolé par filtration ou centrifugation. Afin d'éliminer les traces de composé de formule (I) n'ayant pas réagi, on peut rincer le pigment à l'eau avant ou après filtration ou centrifugation.

Dans le cas où l'on met en oeuvre un procédé de polymérisation oxydante à l'air, il est également possible d'isoler le pigment par lyophilisation.

On obtient ainsi un pigment ayant une granulométrie de 100-150 »m qui est alors broyé par voie classique comme indiqué ci-dessus jusqu'à ce que sa granulométrie soit de l'ordre de 15 à 25 »m.

Selon l'invention, les composés utilisés dans le milieu de broyage en tant que milieu liquide constituent également le milieu dans lequel est dispersé le pigment mélanique finement broyé. Lorsqu'il s'agit de liquides, ils sont choisis parmi les composés cités ci-dessus, et de préférence :
- pour les mono-alcools linéaires ou ramifiés, parmi le dodécanol ou l'alcool oléique;
- pour les polyols linéaires ou ramifiés, parmi le propylèneglycol ou la glycérine;
- pour les alcools cyclo-aliphatiques, on choisira le cyclohexanol;
- pour les alcools aromatiques, parmi l'alcool benzylique, l'alcool phényléthylique;
- pour les esters, parmi les myristate et palmitate d'isopropyle, les triglycérides d'acides gras en C₈-C₁₂ tels que le produit dénommé MIGLYOL, la trioléine, le stéarate d'octyle, les benzoates d'alcools gras tels que le produit dénommé FINSOLV, vendu par la Société FINETEX, le tétracaprylate/caprate de pentaérythritol, le tétraisostéarate de pentaérythritol;
- pour les silicones, parmi les polyorganosiloxanes se présentant sous forme d'huile. Ce sont plus particulièrement des polyalkylsiloxanes, des polyarylsiloxanes, des polyalkylarylsiloxanes, des gommes et résines de silicone en mélange avec des silicones volatiles, des polyorganosiloxanes modifiés par des groupements oxyéthylénés et/ou oxypropylénés, les cyclométhicones.

Lorsque le milieu est constitué par une émulsion, les liquides sont choisis, de préférence, parmi ceux cités ci-dessus à l'exclusion des polyols linéaires ou ramifiés, d'un ou plusieurs émulsionnants et d'eau. Les émulsionnants sont bien connus de l'état de la technique. A titre de référence, on peut citer ceux mentionnés dans l'ouvrage Harry's Cosmeticology - 7ème édition, édité par J.B. Wilkinson et R.J. Moore - pages 626 à 638.

Selon une réalisation préférée, lorsque le milieu de broyage est une émulsion, la teneur en phase aqueuse de celle-ci n'excède pas 50% en poids par rapport au poids total de l'émulsion et en particulier n'excède pas 30%.

Le pigment mélanique est généralement présent dans la dispersion finale obtenue dans des concentrations comprises entre 5 et 50% en poids et de préférence entre 15 et 35% en poids par rapport au poids total de la composition.

La composition qui constitue un objet de l'invention se présente sous forme d'une dispersion, comportant au moins 75% de particules ayant une granulométrie inférieure à 10 »m et de préférence au moins 85% de ces particules ont une granulométrie inférieure à 5 »m, dans le milieu défini ci-dessus.

Cette dispersion est particulièrement stable dans le temps, en particulier sa granulométrie reste constante. On n'observe pas de phénomène de réagglomération.

La composition présente par ailleurs l'avantage de pouvoir être utilisée directement et telle quelle dans des compositions cosmétiques.

Cette dispersion peut constituer le pigment noir prépondérant dans les compositions cosmétiques.

Elle peut plus particulièrement être utilisée dans les compositions de maquillage (mascaras, eye-liners, fards à paupières ou fards à joues, fonds de teint, rouges à lèvres, vernis à ongles, poudres), dans des compositions solaires ou dans des compositions de coloration des cheveux.

Ces compositions peuvent se présenter sous forme de lotions plus ou moins épaissies, d'émulsions (lait ou crème), de stick ou de poudre. Dans ces compositions, la concentration en pigments mélaniques, dont au moins 75% des particules ont une granulométrie inférieure à 10 »m et de préférence 85% ont une granulométrie inférieure à 5 »m, est comprise entre 0,001 et 20% en poids.

La dilution de la dispersion de pigment mélanique définie ci-dessus, même dans un milieu aqueux, n'en modifie pas sa qualité en ce sens que la granulométrie reste sensiblement constante sans qu'il y ait de réagglomération et/ou de sédimentation.

Lorsque les compositions sont utilisées pour le maquillage de la peau, des cils et des sourcils, elles peuvent se présenter sous forme solide ou pâteuse, anhydre ou aqueuse et en particulier sous forme de dispersions lipophile, hydrophile ou hydrophile/lipophile, contenant dans l'une ou l'autre des phases ou dans les deux, les particules définies ci-dessus.

Ces compositions présentent l'avantage d'être particulièrement stables et de présenter une bonne innocuité.

Lorsque les compositions sont utilisées pour la protection de l'épiderme humain contre le rayonnement UV, elles constituent des compositions dites "solaires" et elles se présentent généralement sous forme de dispersions dans des solvants ou des corps gras, ou encore sous forme d'émulsions telles que crèmes et laits, de pommades, de gels, de bâtonnets solides ou de mousses aérosols.

Lorsqu'elles sont utilisées sous forme d'émulsions, elles peuvent contenir en outre des agents tensio-actifs bien connus dans l'état de la technique, tels que des agents tensio-actifs anioniques, non-ioniques, cationiques ou amphotères.

Les compositions de maquillage et les compositions solaires peuvent également contenir des corps gras, des solvants organiques, des silicones, des épaississants, des adoucissants, des filtres solaires, des agents anti-mousses, des agents hydratants, des parfums, des conservateurs, des agents anti-oxydants, des charges, des séquestrants, des agents de traitement tels que des polymères anioniques, cationiques, non-ioniques, amphotères ou leurs mélanges, des propulseurs, des agents alcalinisants ou acidifiants, en plus du milieu utilisé pour le broyage et qui constitue le milieu de la dispersion.

Les corps gras peuvent être constitués par une huile ou une cire ou leur mélange, les acides gras, les alcools gras, la vaseline, la paraffine, la lanoline, la lanoline hydrogénée, la lanoline acétylée.

Les huiles sont en particulier choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin.

Les cires sont choisies notamment parmi les cires animales, fossiles, végétales, minérales ou de synthèse et on peut citer les cires d'abeilles, les cires de Carnauba, de Candellila, de canne à sucre, du Japon, les ozokérites, la cire de Montan, les cires microcristallines, les paraffines, les cires de silicone.

Les compositions conformes à l'invention peuvent également contenir en plus des pigments mélaniques, d'autres pigments généralement utilisés en cosmétique, notamment des pigments nacrés et/ou nacrants permettant de varier les colorations susceptibles d'être obtenues ou d'augmenter la protection vis-à-vis du rayonnement ultraviolet. Dans ce dernier cas, on utilise des pigments métalliques tels que les oxydes de titane, de zinc, de cérium ou de zirconium.

On utilise préférentiellement des "nanopigments", de granulométrie inférieure à 100 nm et de préférence comprise entre 5 et 50 nm. Les nanopigments peuvent être enrobés ou non enrobés.

L'invention a également pour objet un procédé de maquillage de la peau, de protection de l'épiderme humain contre les effets néfastes des rayonnements UV ou de coloration des cheveux, mettant en oeuvre les compositions comportant au moins en partie la dispersion de pigments mélaniques telle que définie ci-dessus.

Les exemples suivants sont destinés à illustrer l'invention sans pour autant présenter un caractère limitatif.

Dans la description qui précède et les exemples qui suivent les dénominations : ALPINE, MYGLYOL, FINETEX, MALVERN, DYNOMILL, FINSOLV, CRODA, KERASOL, SPAN, ICI, VISCOATEX, COATEX, CELQUAT, NATIONAL STARCH, DERMACRYL, SYNNOWAX, HENKEL, PARSOL, GIVAUDAN sont à la connaissance de la demanderesse des marques déposées.

### EXEMPLES DE PREPARATION DE LA DISPERSION

### EXEMPLE DE PREPARATION 1

### A) PREPARATION DU PIGMENT MELANIQUE SYNTHETIQUE

On dissout, dans 5 litres d'eau, 500 g de 5,6-dihydroxyindole. On ajoute 5 ml d'ammnoniaque en solution à 20% et on porte le tout à 80°C. On additionne à cette température le mélange constitué de 390 ml d'eau et de 470 g d'une solution de peroxyde d'hydrogène à 50%, en deux heures. Le chauffage est maintenu deux heures supplémentaires après la fin de l'addition.

Le précipité noir est essoré puis lavé à l'eau. Le produit est séché sous vide. On isole 498 g de pigment.

### B)PRE-BROYAGE

Le pigment préparé plus haut A) se présente sous forme de particules grossières allant jusqu'à un diamètre de 600 »m.

Ce pigment dans un premier temps est broyé tel quel dans un broyeur à broche de type ALPINE, type 63 C.

A la sortie du broyeur, le pigment présente une courbe de distribution de taille des particules centrée aux environs de 20 »m, suivant des mesures faites sur un appareil MALVERN Mastersizer SB-OB.

Le pigment est alors prêt au broyage.

### C)PREPARATION DE LA DISPERSION

Le pigment pré-broyé, suivant l'étape B), est broyé dans un broyeur de type DYNOMILL-KDL-Spécial, dont les billes sont en oxyde de zirconium de diamètre 1 à 1,25 mm.

La charge pour le broyage est composée de :

| | |
|---|---|
| - Pigment | 300 g |
| - Phase liquide | 700 g |
| - Billes d'oxyde de zirconium | 1 kg |

Le broyage est conduit à la température ambiante, pendant 7 heures, dans les différents milieux indiqués ci-après.

| EXEMPLES DE PREPARATIONS | 1 | 2 |
|---|---|---|
| PIGMENT OBTENU A L'ETAPE B | 30 | 30 |
| PROPYLENEGLYCOL | 70 | |
| TRIGLYCERIDE D'ACIDE CAPRIQUE-CAPRYLIQUE VENDU PAR HULS SOUS LA DENOMINATION MIGLYOL 812 | | 70 |

Ces compositions sont particulièrement stables au stockage.

### EXEMPLE DE PREPARATION 3

On opère comme pour les exemples 1 et 2 ci-dessus en utilisant comme liquide de dispersion de l'huile de vaseline. On obtient une dispersion fine et stable de couleur noire.

### EXEMPLE DE PREPARATION 4

On mélange 30% de pigment obtenu à l'étape B) de l'exemple de préparation 1 avec 70% de silicone (décaméthylpentacyclosiloxane).

70% de la préparation précédente sont mélangés à 30% de billes d'oxyde de zirconium.

Le broyage est conduit à température ambiante pendant 5 heures.

On obtient une dispersion fine et régulière de couleur noire.

### EXEMPLE DE PREPARATION 5

On opère comme pour l'exemple 4, en remplaçant l'huile de silicone par le mélange : FINSOLV TN*/HEXYLENE GLYCOL 30/70.
* FINSOLV TN = benzoates d'alcools gras en C₁₂-C₁₅.

La dispersion obtenue est fine et stable, de couleur noire.

### EXEMPLE DE PREPARATION 6

On opère comme dans les exemples 1 à 3, en utilisant comme mélange de dispersion, l'émulsion obtenue par mélange de :

| | |
|---|---|
| - Miglyol 812 | 90 g |
| - Eau | 21 g |
| - Monostéarate de sorbitol (SPAN 60 vendu par ICI) | 4,2 g |
| - Alcool cétylstéarylique oxyéthyléné à 15 moles d'oxyde d'éthylène | 4,2 g |

On obtient une dispersion fine et stable de couleur noire.

### EXEMPLE D'APPLICATION 1

### PREPARATION D'UN MASCARA

La composition du mascara est :

| | |
|---|---|
| - Stéarate de triéthanolamine | 7 g |
| - Cire d'abeilles | 4 g |
| - Cire de carnauba | 1 g |
| - Paraffine | 6,5 g |
| - Colophane | 0,7 g |
| - Dispersion de pigment mélanique dans une phase liquide obtenue selon l'étape C) de l'exemple de préparation 1 | 4,7 g |
| - Hydrolysat de kératine, vendu par CRODA sous la dénomination KERASOL | 2,0 g |
| - Gomme arabique | 0,5 g |
| - Conservateur qs | |
| - Eau purifiée qsp | 100 g |

Le stéarate de triéthanolamine, les cires, la paraffine et la colophane sont chauffés à 85°C, puis on y introduit la dispersion du pigment mélanique.

La phase aqueuse contenant le reste de la formule est chauffée séparément à 85°C, puis les deux phases sont mélangées.

Pour ces préparations, on utilise les dispersions de pigment préparées selon les exemples 1 à 3 ci-dessus.

L'exemple 1a) contient la dispersion mélanique dans le propylène glycol de l'exemple de préparation 1.

L'exemple 1b) contient la dispersion de pigment mélanique dans le Miglyol 812 de l'exemple de préparation 2.

Les mascaras des exemples 1a) et 1b) sont de couleur noire. L'examen au microscope met en évidence une dispersion fine et régulière. Le maquillage des cils est facile. Les cils sont épaissis et bien séparés. Le maquillage présente une bonne tenue.

### EXEMPLE D'APPLICATION 2

### PREPARATION D'UN MASCARA

On prépare la composition suivante :

| | |
|---|---|
| - Cire de Carnauba | 5 g |
| - Cire de Candelilla | 5 g |
| - Alcool éthylique | 3 g |
| - Montmorillonite | 6 g |
| - Dispersion à 25% d'un pigment mélanique dispersé dans l'huile de vaseline selon l'exemple de préparation 3 | 3,5 g |
| - Talc | 10 g |
| - Isoparaffine qsp | 100 g |

Le mode opératoire est le suivant :

On chauffe les cires à 80°C. On ajoute le talc et la dispersion de pigments mélaniques. On incorpore ensuite la Montmorillonite et une partie de l'isoparaffine. A environ 40°C, on introduit l'alcool éthylique et le reste de l'isoparaffine. On passe le tout à la broyeuse. Dans ce cas, on obtient un mascara dit "waterproof" (résistant à l'eau).

### EXEMPLE D'APPLICATION 3

Dans l'exemple d'application 2, la dispersion préparée selon l'exemple 3 peut être remplacée par la dispersion préparée selon l'exemple 6.

### EXEMPLE D'APPLICATION 4

### FOND DE TEINT

On prépare le composition suivante :

| | |
|---|---|
| - Propylèneglycol | 2 g |
| - Magnésium aluminium | 1 g |
| - Carboxyméthyl cellulose | 0,2 g |
| - Triéthanolamine | 1 g |
| - Acide stéarique | 2,2 g |
| - Stéarate de glycéryle | 2,2 g |
| - Cyclométhicone | 10 g |
| - Paradiméthylaminobenzoate de 2-éthylhexyle | 0,5 g |
| - 2-hydroxy 4-méthoxybenzophénone | 0,5 g |
| - Produit de l'exemple de préparation 3 | 1,5 g |
| - Glycérine | 3 g |
| - Lauryl sarcosinate de sodium | 0,6 g |
| - Pigments | 10 g |
| - Triglycéride d'acides caprilique/caprique | 15 g |
| - Conservateurs | 0,5 g |
| - Eau qsp | 100 g |

La composition se présente sous forme d'une dispersion fine et régulière, stable après 2 mois de stockage.

### EXEMPLE D'APPLICATION 5

### GEL DE MAQUILLAGE DES CHEVEUX

On prépare la composition suivante :

| | |
|---|---|
| - Copolymère acide méthacrylique/acrylate d'éthyle réticulé en dispersion anionique à 38% de MA, vendu sous la dénomination VISCOATEX 538 par la Société COATEX | 3,95 g MA |
| - Copolymère hydroxyéthylcellulose/chlorure de diallyl diméthyl ammonium, vendu sous la dénomination CELQUAT LOR par la Société NATIONAL STARCH | 1,5 g |
| - Ethanol | 10 g |
| - Copolymère acrylate/t-octylpropenamide, vendu sous la dénomination DERMACRYL 79 par la Société NATIONAL STARCH | 1,5 g |
| - Stéaryl diméthylamine | 3,75 g |
| - Pigment mélanique broyé dans le propylène glycol à 20% de MA | 2,5 g MA |
| - 2-amino 2-méthyl 1-propanol pH=7.5 | |
| - Conservateur, séquestrant, parfum qs | |
| - Eau qsp | 100 g |

### EXEMPLE D'APPLICATION 6

### COMPOSITION SOLAIRE

| | |
|---|---|
| - Mélange d'alcool cétylstéarylique et d'alcool cétylstéarylique 33 OE, vendu sous la dénomination SYNNOWAX AO par la Société HENKEL | 7 g |
| - Mélange de monostéarate et de distéarate de glycéryle non auto-émulsionnable | 2 g |
| - Alcool cétylique | 1,5 g |
| - Huile de silicone | 1,5 g |
| - Huile de vaseline | 7 g |
| - Dispersion de pigment mélanique obtenue selon l'exemple 3, diluée dans l'huile de vaseline (1,25% en poids de pigment mélanique) | 8 g |
| - Paraméthoxycinnamate de 2-éthylhexyle, vendu sous la dénomination PARSOL MCX par la Société GIVAUDAN | 5 g |
| - Glycérine | 20 g |
| - Parfum, conservateurs qs | |
| - Eau qsp | 100 g |

## Revendications

1. Dispersion de pigments mélaniques, caractérisée par le fait qu'elle contient dans un milieu choisi parmi :
a) les liquides choisis parmi :
- les mono-alcools linéaires ou ramifiés comportant au moins 3 atomes de carbone :
- les polyols linéaires ou ramifiés comportant au moins 2 atomes de carbone;
- les alcools cycloaliphatiques saturés ou insaturés comportant au moins 6 atomes de carbone;
- les alcools aromatiques comportant au moins 6 atomes de carbone;
- les esters d'acides gras en C₈-C₂₂ et d'alcools en C₁-C₆ ou de polyols en C₂-C₆; les esters d'acides aliphatiques en C₁-C₆ ou d'acides aromatiques et d'alcools gras en C₈-C₂₂; les esters d'acides gras en C₈-C₂₂ et d'alcools gras en C₈-C₂₂, les esters de polyacides et d'alcools en C₆-C₂₂, les diesters d'acides gras hydroxylés;
- les paraffines linéaires ou ramifiées comportant au moins 8 atomes de carbone;
- les huiles de silicones;
et le mélange de ces différents composés,
ou
b) une émulsion constituée par un liquide défini ci-dessus, non miscible à l'eau, de l'eau et un agent émulsionnant,
un pigment mélanique sous forme de particules dont au moins 75% des particules ont une granulométrie inférieure à 10 »m.

2. Dispersion selon la revendication 1, caractérisée par le fait qu'au moins 85% des particules ont une granulométrie inférieure à 5 »m.

3. Dispersion selon la revendication 1 ou 2, caractérisée par le fait que la concentration en pigments mélaniques dans la dispersion est comprise entre 5 et 50% en poids et de préférence entre 15 et 35% en poids par rapport au poids total de la composition.

4. Procédé de préparation d'une dispersion selon l'une quelconque des revendications 1 à 3, caractérisé par le fait que l'on introduit dans un milieu liquide choisi parmi :
a) un composé choisi parmi :
- un mono-alcool linéaire ou ramifié, comportant au moins 3 atomes de carbone;
- un polyol linéaire ou ramifié, comportant au moins 2 atomes de carbone;
- un alcool cycloaliphatique saturé ou insaturé, comportant au moins 6 atomes de carbone;
- un alcool aromatique, comportant au moins 6 atomes de carbone;
- un ester d'acide gras en C₈-C₂₂ et d'alcool en C₁-C₆ ou de polyol en C₂-C₆; un ester d'acide aliphatique en C₁-C₆ ou d'acide aromatique et d'alcool gras en C₈-C₂₂; un ester d'acide gras en C₈-C₂₂ et d'alcool gras en C₈-C₂₂, un ester de polyacide et d'alcool en C₆-C₂₂, un diester d'acide gras hydroxylé;
- une paraffine linéaire ou ramifiée comportant au moins 8 atomes de carbone;
- une huile de silicone;
et le mélange de ces différents composés, ou
b) une émulsion constituée par les liquides définis sous a), non miscibles à l'eau, de l'eau et un agent émulsionnant,
un pigment mélanique d'origine naturelle ou synthétique et que l'on procède au broyage en milieu liquide jusqu'à ce qu'au moins 75% des particules aient une granulométrie inférieure à 10 »m et de préférence 85% des particules aient une granulométrie inférieure à 5 »m.

5. Procédé selon la revendication 4, caractérisé par le fait que le pigment mélanique introduit dans le milieu de broyage a une granulométrie comprise entre 15 et 25 »m.

6. Procédé selon la revendication 4 ou 5, caractérisé par le fait que l'on utilise un pigment mélanique d'origine naturelle ou synthétique.

7. Procédé selon la revendication 6, caractérisé par le fait que l'on utilise comme pigment mélanique un pigment préparé par polymérisation oxydante d'un composé indolique répondant à la formule (I) : 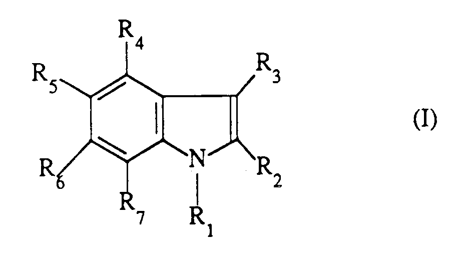 dans laquelle :
R₁ et R₃ représentent, indépendamment l'un de l'autre, un atome d'hydrogène ou un groupe alkyle en C₁-C₄;
R₂ représente un atome d'hydrogène, un groupe alkyle en C₁-C₄, un groupe carboxyle ou un groupe alcoxy(C₁-C₄)carbonyle;
R₄ et R₇ représentent, indépendamment l'un de l'autre, un atome d'hydrogène, un groupe hydroxy, un groupe alkyle en C₁-C₄, un groupe amino, un groupe alcoxy en C₁-C₄, un groupe acyl(C₂-C₄)oxy ou un groupe acyl(C₂-C₄)amino;
R₅ représente un atome d'hydrogène, un groupe hydroxy, un groupe alcoxy en C₁-C₄, un groupe alkyle en C₁-C₄, un atome d'halogène, un groupe amino, un groupe acyl(C₂-C₁₄)oxy, un groupe acyl(C₂-C₄)amino ou un groupe triméthylsilyloxy;
R₆ représente un atome d'hydrogène, un groupe hydroxy, un groupe alcoxy en C₁-C₄, un groupe amino, un groupe acyl(C₂-C₄)oxy, un groupe acyl(C₂-C₄)amino, un groupe triméthylsilyloxy ou un groupe hydroxyalkyl(C₂-C₄)amino;
R₅ et R₆ pouvant également former, conjointement avec les atomes de carbone auxquels ils sont rattachés, un cycle carbonyldioxy;
au moins l'un des radicaux R₄ à R₇ représente un groupement OZ ou NHR₈, l'un au plus des radicaux R₄ à R₇ représentant NHR₈ et deux au plus des radicaux R₄ à R₇ représentant OZ et, dans le cas où Z représente un atome d'hydrogène, les deux groupes OH sont dans les positions 5 et 6; et au moins l'un des radicaux R₄ à R₇ représente un atome d'hydrogène, et dans le cas où un seul de ces radicaux R₄ à R₇ représente un atome d'hydrogène, alors un seul radical parmi R₄ à R₇ représente NHR₈ ou OZ, les autres radicaux représentant un groupe alkyle en C₁-C₄;
le radical R₈ du groupement NHR₈ désignant un atome d'hydrogène, un groupe acyle en C₂-C₄ ou hydroxyalkyle en C₂-C₄, et le radical Z du groupement OZ désignant un atome d'hydrogène, un groupe acyle en C₂-C₁₄, un groupe alkyle en C₁-C₄, ou un groupe triméthylsilyle,
et leurs sels de métaux alcalins, alcalino-terreux, d'ammonium ou d'amines.

8. Procédé selon la revendication 7, caractérisé par le fait que les composés indoliques de formule (I) sont choisis parmi :
le 4-hydroxyindole, le 5-hydroxyindole, le 6-hydroxyindole, le 7-hydroxyindole, le 4-hydroxy 5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthylindole, le 2-carboxy 6-hydroxyindole, le 6-hydroxy N-méthyl indole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N,β-hydroxyéthyl aminoindole, le 4-aminoindole, le 5-aminoindole, le 6-aminoindole, le 7-aminoindole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-triméthylsilyloxyindole, l'ester phosphorique du 5,6-dihydroxyindole, le 5,6-dibenzyloxyindole, et les sels d'addition de ces composés.

9. Procédé selon l'une quelconque des revendications 4 à 8, caractérisé par le fait que l'on introduit la dispersion contenant le pigment mélanique à broyer dans un broyeur à billes.

10. Procédé selon l'une quelconque des revendications 4 à 9, caractérisé par le fait qu'on utilise un broyeur à billes utilisant des billes d'oxyde de zirconium, des billes de verte ou du sable.

11. Procédé selon l'une quelconque des revendications 4 à 10, caractérisé par le fait que la composition soumise au broyage contient 5 à 50% en poids par rapport au poids total de la composition de pigments mélaniques.

12. Procédé selon l'une quelconque des revendications 4 à 11, caractérisé par le fait que la température de broyage est comprise entre la température ambiante et 110°C suivant que le milieu utilisé pour le broyage est solide ou non à température ambiante.

13. Procédé selon l'une quelconque des revendications 4 à 12, caractérisé par le fait que les mono-alcools linéaires ou ramifiés sont choisis parmi le dodécanol, l'alcool oléique; les polyols linéaires ou ramifiés sont choisis parmi le propylèneglycol ou la glycérine; l'alcool cycloaliphatique est le cyclohexanol; les alcools aromatiques sont choisis parmi l'alcool benzylique ou l'alcool phényléthylique; les esters sont choisis parmi les myristate et palmitate d'isopropyle, les triglycérides d'acides gras en C₈-C₁₂, la trioléine, le stéarate d'octyle, les benzoates d'alcools gras, le tétracaprylate/caprate de pentaérythritol, le tétraisostéarate de pentaérythritol; les silicones sont des polyorganosiloxanes se présentant sous forme d'huile.

14. Procédé selon la revendication 13, caractérisé par le fait que les silicones sont des polyalkylsiloxanes, des polyarylsiloxanes, des polyalkylarylsiloxanes, des gommes et résines de silicone en mélange avec des silicones volatiles, des polyorganosiloxanes modifiés par des groupements oxyéthylénés et/ou oxypropylénés.

15. Dispersion obtenue par la mise en oeuvre du procédé tel que défini dans l'une quelconque des revendications 4 à 14.

16. Utilisation de la dispersion selon l'une quelconque des revendications 1 à 3 et 15, pour la préparation de compositions de maquillage, de compositions solaires ou de compositions de coloration des cheveux.

17. Composition cosmétique, caractérisée par le fait qu'elle comprend au moins en partie une dispersion telle que définie dans l'une quelconque des revendications 1 à 3 ou 15, dans un milieu cosmétiquement acceptable.

18. Composition selon la revendication 17, caractérisée par le fait qu'elle comprend des pigments mélaniques sous forme de particules dont au moins 75% des particules ont une granulomérie inférieure à 10 »m et de préférence 85% ont une granulométrie inférieure à 5 »m dans des proportions comprises entre 0,001 et 20% en poids par rapport au poids total de la composition.

19. Composition selon l'une quelconque des revendications 17 et 18, destinée à être utilisée pour le maquillage de la peau, des cils et des sourcils, caractérisée par le fait qu'elle se présente sous forme solide ou pâteuse, anhydre ou aqueuse.

20. Composition selon l'une quelconque des revendications 17 et 18, destinée à la protection de l'épiderme humain contre le rayonnement UV, caractérisée par le fait qu'elle se présente sous forme de suspension dans des solvants ou des corps gras, sous forme d'émulsions, de pommades, de gels, de bâtonnets solides, de mousse aérosols.

21. Composition selon l'une quelconque des revendications 17 et 18, caractérisée par le fait qu'elle contient des corps gras, des solvants organiques, des silicones, des épaississants, des adoucissants, des tensio-actifs, des filtres solaires, des agents anti-mousses, des agents hydratants, des parfums, des conservateurs, des agents anti-oxydants, des charges, des séquestrants, des agents de traitement, des propulseurs, des agents alcalinisants, acidifiants ou d'autres pigments.

22. Composition selon la revendication 20, caractérisée par le fait qu'elle contient un nanopigment d'oxyde métallique choisi parmi les oxydes de titane, de zinc, de cérium ou de zirconium, ces nanopigments ayant un diamètre moyen inférieur à 100 nm et sont enrobés ou non-enrobés.

## Claims

1. Dispersion of melanin pigments, characterized in that it contains, in a medium chosen from:
a) liquids chosen from:
- linear or branched monoalcohols containing at least 3 carbon atoms;
- linear or branched polyols containing at least 2 carbon atoms;
- saturated or unsaturated cycloaliphatic alcohols containing at least 6 carbon atoms;
- aromatic alcohols containing at least 6 carbon atoms;
- esters of C₈-C₂₂ fatty acids and C₁-C₆ alcohols or C₂-C₆ polyols; esters of C₁-C₆ aliphatic acids or aromatic acids and C₈-C₂₂ fatty alcohols; esters of C₈-C₂₂ fatty acids and C₈-C₂₂ fatty alcohols, esters of C₆-C₂₂ alcohols and polyacids, and diesters of hydroxylated fatty acids;
- linear or branched paraffins containing at least 8 carbon atoms;
- silicone oils;
- and the mixture of these various compounds, or
b) an emulsion consisting of a water-immiscible liquid defined above, water and an emulsifying agent,
a melanin pigment in the form of particles, for which at least 75 % of the particles have a particle size lower than 10 »m.

2. Dispersion according to claim 1, characterized in that at least 85 % of the particles have a particle size lower than 5 »m.

3. Dispersion according to claim 1 or 2, characterized in that the concentration of melanin pigments in the dispersion is between 5 and 50 % by weight, and preferably between 15 and 35 % by weight relative to the total weight of the composition.

4. Process for the preparation of a dispersion according to any one of claims 1 to 3, characterized in that, into a liquid medium chosen from:
a) a liquid chosen from:
- a linear or branched monoalcohol containing at least 3 carbon atoms;
- a linear or branched polyol containing at least 2 carbon atoms;
- a saturated or unsaturated cycloaliphatic alcohol containing at least 6 carbon atoms;
- an aromatic alcohol containing at least 6 carbon atoms;
- an ester of a C₈-C₂₂ fatty acid and a C₁-C₆ alcohol or a C₂-C₆ polyol, an ester of a C₁-C₆ aliphatic acid or an aromatic acid and a C₈-C₂₂ fatty alcohol, an ester of a C₈-C₂₂ fatty acid and a C₈-C₂₂ fatty alcohol, an ester of a polyacid and a C₆-C₂₂ alcohol, a diester of a hydroxylated fatty acid;
- a linear or branched paraffin containing at least 8 carbon atoms;
- a silicone oil;
and the mixture of these various compounds, or
b) an emulsion consisting of a water-immiscible liquid defined above, water and an emulsifying agent,
there is introduced a melanin pigment of natural or synthetic origin and in that grinding is performed in a liquid medium until at least 75 % of the particles have a particle size lower than 10 »m and preferably 85 % of the particles have a particle size lower than 5 »m.

5. Process according to claim 4, characterized in that the melanin pigment introduced into the grinding medium has a particle size between 15 and 25 »m.

6. Process according to claim 4 or 5, characterized in that a melanin pigment of natural or synthetic origin is used.

7. Process according to claim 6, characterized in that the melanin pigment used is a pigment prepared by oxidative polymerization of an indole compound corresponding to the formula (I): 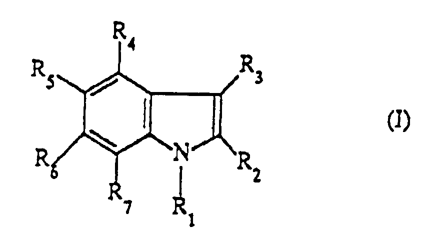 in which:
R₁ and R₃ represent, independently of each other, a hydrogen atom or a C₁-C₄ alkyl group;
R₂ represents a hydrogen atom, a C₁-C₄ alkyl group, a carboxyl group or a (C₁-C₄ alkoxy)carbonyl group;
R₄ and R₇ represent, independently of each other, a hydrogen atom, a hydroxyl group, a C₁-C₄ alkyl group, an amino group, a C₁-C₄ alkoxy group, a (C₂-C₄ acyl)oxy group or a (C₂-C₄ acyl)amino group;
R₅ represents a hydrogen atom, a hydroxyl group, a C₁-C₄ alkoxy group, a C₁-C₄ alkyl group, a halogen atom, an amino group, a (C₂-C₁₄ acyl)oxy group, a (C₂-C₄ acyl)amino group or a trimethylsilyloxy group;
R₆ represents a hydrogen atom, a hydroxyl group, a C₁-C₄ alkoxy group, an amino group, a (C₂-C₄ acyl)oxy group, a (C₂-C₄ acyl)amino group, a trimethylsilyloxy group or a (C₂-c₄ hydroxyalkyl)amino group;
it is also possible for R₅ and R₆ to form, together with the carbon atoms to which they are attached, a carbonyldioxy ring;
at least one of the radicals R₄ to R₇ represents a group OZ or NHR₈, no more than one of the radicals R₄ to R₇ representing NHR₈ and no more than two of the radicals R₄ to R₇ representing OZ and, in the case where Z represents a hydrogen atom, the two OH groups are in positions 5 and 6; and at least one of the radicals R₄ to R₇ represents a hydrogen atom, and in the case where only one of these radicals R₄ to R₇ represents a hydrogen atom, then only one radical among R₄ to R₇ represents NHR₈ or OZ, the other radicals representing a C₁-C₄ alkyl group;
the radical R₈ of the group NHR₈ denoting a hydrogen atom, a C₂-C₄ acyl or C₂-C₄ hydroxyalkyl group, and the radical Z of the group OZ denoting a hydrogen atom, a C₂-C₁₄ acyl group, a C₁-C₄ alkyl group or a trimethylsilyl group,
and their salts with alkali metals, alkaline-earth metals, ammonium or amines.

8. Process according to claim 7, characterized in that the indole compounds of formula (I) are chosen from: 4-hydroxyindole, 5-hydroxyindole, 6-hydroxyindole, 7-hydroxyindole, 4-hydroxy-5-methoxyindole, 4-hydroxy-5-ethoxyindole, 2-carboxy-5-hydroxyindole, 5-hydroxy-6-methoxyindole, 6-hydroxy-7-methoxyindole, 5-methoxy-6-hydroxyindole, 5,6-dihydroxyindole, N-methyl-5,6-dihydroxyindole, 2-methyl-5,6-dihydroxyindole, 3-methyl-5,6-dihydroxyindole, 2,3-dimethyl-5,6-dihydroxyindole, 2-carboxy-5,6-dihydroxyindole, 4-hydroxy-5-methylindole, 2-carboxy-6-hydroxyindole, 6-hydroxy-N-methylindole, 2-ethoxycarbonyl-5,6-dihydroxyindole, 4-hydroxy-7-methoxy-2,3-dimethylindole, 4-hydroxy-5-ethoxy-N-methylindole, 6-hydroxy-5-methoxy-2-methylindole, 6-hydroxy-5-methoxy-2,3-dimethylindole, 6-hydroxy-2-ethoxycarbonylindole, 7-hydroxy-3-methylindole, 5-hydroxy-6-ethoxy-2,3-dimethyl-indole, 5-hydroxy-3-methylindole, 5-acetoxy-6-hydroxyindole, 5-hydroxy-2-ethoxycarbonylindole, 6-hydroxy-2-carboxy-5-methylindole, 6-hydroxy-2-ethoxycarbonyl-5-methoxyindole, 6-N-β-hydroxyethylaminoindole [sic], 4-aminoindole, 5-aminoindole, 6-aminoindole, 7-aminoindole, N-methyl-6-β-hydroxyethylaminoindole, 6-amino-2,3-dimethylindole, 6-aminoindole-2,3,4,5-tetramethylindole, 6-amino-2,3,4-trimethylindole, 6-amino-2,3,5-trimethylindole, 6-amino-2,3,6-trimethylindole, 5,6-diacetoxyindole, 5-methoxy-6-acetoxyindole, 5,6-trimethylsilyloxyindole [sic], the phosphoric ester of 5,6-dihydroxyindole, 5,6-dibenzyloxyindole, and the addition salts of these compounds.

9. Process according to any one of claims 4 to 8, characterized in that the dispersion containing the melanin pigment to be ground is introduced into a grinder containing beads.

10. Process according to any one of claims 4 to 9, characterized in that a grinder containing beads using zirconium oxide beads, glass beads or sand is used.

11. Process according to any one of claims 4 to 10, characterized in that the composition subjected to the grinding contains 5 to 50 % by weight of melanin pigments relative to the total weight of the composition.

12. Process according to any one of claims 4 to 11, characterized in that the grinding temperature is between room temperature and 110°C depending on whether the medium used for the grinding is solid or otherwise at room temperature.

13. Process according to any one of claims 4 to 12, characterized in that the linear or branched monoalcohols are chosen from dodecanol and oleyl alcohol; the linear or branched polyols are chosen from propylene glycol or glycerine; the cycloaliphatic alcohol is cyclohexanol; the aromatic alcohols are chosen from benzyl alcohol or phenylethyl alcohol; the esters are chosen from isopropyl myristate and palmitate, c₈-C₁₂ fatty acid triglycerides, triolein, octyl stearate, fatty alcohol benzoates, pentaerythritol caprylate/caprate or pentaerythritol tetraisostearate; the silicones are polyorganosiloxanes in oil form.

14. Process according to claim 13, characterized in that the silicones are polyalkylsiloxanes, polyarylsiloxanes, polyalkylarylsiloxanes, silicone gums and resins mixed with volatile silicones, polyorganosiloxanes modified by oxyethylenated and/or oxypropylenated groups.

15. Dispersion obtained by the implementation of the process as defined in any one of claims 4 to 14.

16. Use of the dispersion according to any one of claims 1 to 3 and 15, for the preparation of make-up compositions, sun care compositions or compositions for coloring hair.

17. Cosmetic composition, characterized in that it comprises, at least in part, a dispersion as defined in any one of claims 1 to 3 or 15, in a cosmetically acceptable medium.

18. Composition according to claim 17, characterized in that it comprises melanin pigments in the form of particles, for which at least 75 % of the particles have a particle size lower than 10 »m and preferably 85 % have a particle size lower than 5 »m, in proportions between 0.001 and 20 % by weight relative to the total weight of the composition.

19. Composition according to either of claims 17 and 18, intended to be used for making up skin, eyelashes and eyebrows, characterized in that it is present in solid or pasty, anhydrous or aqueous form.

20. Composition according to either of claims 17 and 18, intended for protecting human skin against UV rays, characterized in that it takes the form of a suspension in solvents or fatty substances, or takes the form of emulsions, ointments, gels, small solid sticks or aerosol foams.

21. Composition according to either of claims 17 and 18, characterized in that it contains contain fatty substances, organic solvents, silicones, thickeners, emollients, sun filters, antifoaming agents, moisturizing agents, perfumes, preservatives, antioxidizing agents, fillers, sequestering agents, treating agents, propellants and basifying or acidifying agents, or other pigments.

22. Composition according to claim 20, characterized in that it contains a nanopigment of metallic oxide chosen from the oxides of titanium, zinc, cerium or zirconium, these nanopigments having a mean diameter lower than 100 nm and are [sic] coated or non-coated.

## Patentansprüche

1. Dispersion von Melanin-Pigmenten,
dadurch **gekennzeichnet**, daß
sie in einem Milieu, ausgewählt aus:
a) einer Flüssigkeit, die aus:
- einem linearen oder verzweigten Monoalkohol mit mindestens 3 Kohlenstoffatomen;
- einem linearen oder verzweigten Polyol mit mindestens 2 Kohlenstoffatomen;
- einem gesättigten oder ungesättigten cycloaliphatischen Alkohol mit mindestens 6 Kohlenstoffatomen;
- einem aromatischen Alkohol mit mindestens 6 Kohlenstoffatomen;
- einem Ester aus einer C₈-₂₂-Fettsäure und einem C₁₋₆-Alkohol oder einem C₂₋₆-Polyol, einem Ester aus einer aliphatischen C₁₋₆-Säure oder einer aromatischen Säure mit einem C₈₋₂₂-Fettalkohol, einem Ester aus einer C₈₋₂₂-Fettsäure mit einem C₈₋₂₂-Fettalkohol, einem Ester aus einer Polysäure mit einem C₆₋₂₂-Alkohol, einem Diester einer hydroxylierten Fettsäure;
- einem linearen oder verzweigten Paraffin mit mindestens 8 Kohlenstoffatomen;
- einem Siliconöl;
und aus einer Mischung dieser verschieden Verbindungen ausgewählt ist,
oder aus
b) einer Emulsion aus einer oben definierten, nicht mit Wasser mischbaren Flüssigkeit, Wasser und einem Emulgator,
ein Melanin-Pigment in Form von Partikeln, wovon mindestens 75% der Partikel eine Korngröße von weniger als 10 »m aufweisen,
enthält.

2. Dispersion gemäß Anspruch 1,
dadurch **gekennzeichnet**, daß
mindestens 85% der Partikel eine Korngröße von weniger als 5 »m aufweisen.

3. Dispersion gemäß Anspruch 1 oder 2,
dadurch **gekennzeichnet**, daß
die Konzentration an Melanin-Pigmenten in der Dispersion 5 bis 50 und vorzugsweise 15 bis 35 Gew.% ausmacht, bezogen auf das Gesamtgewicht der Zusammensetzung.

4. Verfahren zur Herstellung einer Dispersion gemäß jedem der Ansprüche 1 bis 3,
dadurch **gekennzeichnet**, daß
man in ein flüssiges Milieu, das aus:
a) einer Verbindung aus:
- einem linearen oder verzweigten Monoalkohol mit mindestens 3 Kohlenstoffatomen;
- einem linearen oder verzweigten Polyol mit mindestens 2 Kohlenstoffatomen;
- einem gesättigten oder ungesättigten cycloaliphatischen Alkohol mit mindestens 6 Kohlenstoffatomen;
- einem aromatischen Alkohol mit mindestens 6 Kohlenstoffatomen;
- einem Ester aus einer C₈₋₂₂-Fettsäure und einem C₁₋₆-Alkohol oder einem C₂₋₆-Polyol, einem Ester aus einer aliphatischen C₁₋₆-Säure oder einer aromatischen Säure mit einem C₈₋₂₂-Fettalkohol, einem Ester aus einer C₈₋₂₂-Fettsäure mit einem C₈₋₂₂-Fettalkohol, einem Ester aus einer Polysäure mit einem C₆₋₂₂-Alkohol, einem Diester einer hydroxylierten Fettsäure;
- einem linearen oder verzweigten Paraffin mit mindestens 8 Kohlenstoffatomen;
- einem Siliconöl;
- und aus einer Mischung dieser verschieden Verbindungen, oder aus
b) einer Emulsion ausgewählt ist, die aus den unter a) definierten, nicht mit Wasser mischbaren Flüssigkeiten, Wasser und einem Emulgator zusammengesetzt ist,
ein Melanin-Pigment natürlichen oder synthetischen Ursprungs einbringt und man in dem flüssigen Milieu eine Verreibung durchführt, bis mindestens 75% der Partikel eine Korngröße von weniger als 10 »m und vorzugsweise 85% der Partikel eine Korngröße von weniger als 5 »m aufweisen.

5. Verfahren gemäß Anspruch 4,
dadurch **gekennzeichnet**, daß
das in das Verreibungsmilieu eingebrachte Melanin-Pigment eine Korngröße von 15 bis 25 »m aufweist.

6. Verfahrn gemäß Anspruch 4 oder 5,
dadurch **gekennzeichnet**, daß
man ein Melanin-Pigment natürlichen oder synthetischen Ursprungs einsetzt.

7. Verfahren gemäß Anspruch 6,
dadurch **gekennzeichnet**, daß
man ein Melanin-Pigment einsetzt, das durch oxidierende Polymerisation einer Indolverbindung der Formel (I): 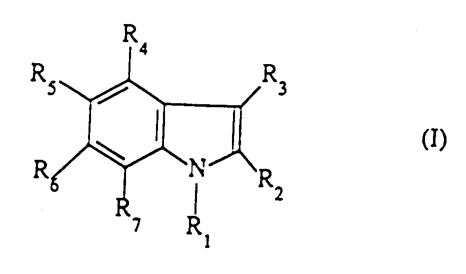 worin gilt: R₁ und R₂ stellen, unabhängig voneinander, ein Wasserstoffatom oder eine C₁₋₄-Alkylgruppe dar;
R₂ stellt ein Wasserstoffatom, eine C₁₋₄-Alkyl-, Carboxyl- oder C₁₋₄-Alkoxycarbonylgruppe dar;
R₄ und R₇ stellen, unabhängig voneinander, ein Wasserstoffatom, eine Hydroxy-, C₁₋₄-Alkyl-, Amino-, C₁₋₄-Alkoxy-, C₂₋₄-Acyloxy oder C₂₋₄-Acylaminogruppe dar;
R₅ stellt ein Wasserstoffatom, eine Hydroxy-, C₁₋₄-Alkoxy-, C₁₋₄-Alkylgruppe, ein Halogenatom, eine Amino-, C₂₋₁₄-Acyloxy-, C₂₋₄-Acylamino- oder eine Trimethylsilyloxygruppe dar;
R₆ stellt ein Wasserstoffatom, eine Hydroxy-, C₁₋₄-Alkoxy-, Amino-, C₂₋₄-Acyloxy-, C₂₋₄-Acylamino-, Trimethylsilyloxy- oder eine Hydroxy-C₂₋₄-alkylaminogruppe dar,
wobei R₅ und R₆ zusammen mit den Kohlenstoffatomen, an die sie gebunden sind, auch einen Carbonyldioxi-Ring bilden können;
mindestens einer der Reste R₄ bis R₇ stellt eine OZ- oder NHR₈-Gruppe dar, wobei höchstens einer der Reste R₄ bis R₇ eine NHR₈- und höchstens zwei der Reste R₄ bis R₇ eine OZ-Gruppe darstellen, und wobei, wenn Z ein Wasserstoffatom darstellt, die beiden OH-Gruppen dann in den Positionen 5 und 6 vorliegen; und mindestens einer der Reste R₄ bis R₇ stellt ein Wasserstoffatom dar, und wenn einer dieser Reste R₄ bis R₇ ein Wasserstoffatom darstellt, stellen nur einer der Reste R₄ bis R₇ eine NHR₈- oder OZ-Gruppe und die weiteren Reste eine C₁₋₄-Alkylgruppe dar;
wobei der Rest R₈ der NHR₈- Gruppe ein Wasserstoffatom, eine C₂₋₄-Acyl- oder Hydroxy-C₂₋₄-Akylgruppe und der Rest Z der OZ-Gruppe ein Wasserstoffatom, eine C₂₋₁₄-Acyl-, C₁₋₄-Alkyl- oder eine Trimethylsilylgruppe darstellen,
sowie von deren Alkali-, Erdalkali-, Ammonium- oder Aminsalzen
hergestellt ist.

8. Verfahren gemäß Anspruch 7,
dadurch **gekennzeichnet**, daß
die Indolverbindungen der Formel (I) aus: 4-Hydroxyindol, 5-Hydroxyindol, 6-Hydroxyindol, 7-Hydroxyindol, 4-Hydroxy-5-methoxyindol, 4-Hydroxy-5-ethoxyindol, 2-Carboxy-5-hydroxyindol, 5-Hydroxy-6-methoxyindol, 6-Hydroxy-7-methoxyindol, 5-Methoxy-6-hydroxyindol, 5,6-Dihydroxyindol, N-Methyl-5,6-dihydroxyindol, 2-Methyl-5,6-dihydroxyindol, 3-Methyl-5,6-dihydroxyindol, 2,3-Dimethyl-5,6-dihydroxyindol, 2-carboxy-5,6-dihydroxyindol, 4-Hydroxy-5-methylindol, 2-Carboxy-6-hydroxyindol, 6-Hydroxy-N-methylindol, 2-Ethoxycarbonyl-5,6-dihydroxyindol, 4-Hydroxy-7-methoxy-2,3-dimethylindol, 4-Hydroxy-5-ethoxy-N-methylindol, 6-Hydroxy-5-methoxy-2-methylindol, 6-Hydroxy-5-methoxy-2,3-dimethylindol, 6-Hydroxy-2-ethoxycarbonylindol, 7-Hydroxy-3-methylindol, 5-Hydroxy-6-methoxy-2,3-dimethylindol, 5-Hydroxy-3-methylindol, 5-Acetoxy-6-hydroxyindol, 5-Hydroxy-2-ethoxycarbonylindol, 6-Hydroxy-2-carboxy-5-methylindol, 6-Hydroxy-2-ethoxycarbonyl-5-methoxyindol, 6-N,β-Hydroxyethylaminoindol, 4-Aminoindol, 5-Aminoindol, 6-Aminoindol, 7-Aminoindol, N-Methyl-6-β-hydroxyethylaminoindol, 6-Amino-2,3-dimethylindol, 6-Amino-2,3,4,5-tetramethylindol, 6-Amino-2,3,4-trimethylindol, 6-Amino-2,3,5-trimethylindol, 5-Amino-2,3,6-trimethylindol, 5,6-Diacetoxyindol, 5-Methoxy-6-acetoxyindol, 5,6-Trimethylsilyloxyindol, Posphorsäureester von 5,6-Dihydroxyindol, 5,6-Dibenzyloxyindol und aus den Additionssalzen dieser Verbindungen
ausgewählt sind.

9. Verfahren gemäß jedem der Ansprüche 4 bis 8,
dadurch **gekennzeichnet**, daß
man die das Melanin-Pigment enthaltende Dispersion einbringt, um sie in einer Kugelmühle zu verreiben.

10. Verfahren gemäß jedem der Ansprüche 4 bis 9,
dadurch **gekennzeichnet**, daß
man eine Kugelmühle heranzieht, in welcher Kugeln aus Zirkonoxid, Kugeln aus Glas oder Sand verwendet werden.

11. Verfahren gemäß jedem der Ansprüche 4 bis 10,
dadurch **gekennzeichnet**, daß
die zu verreibende Zusammensetzung 5 bis 50 Gew.% Melanin-Pigmente enthält, bezogen auf das Gesamtgewicht der Zusammensetzung.

12. Verfahren gemäß jedem der Ansprüche 4 bis 11,
dadurch **gekennzeichnet**, daß
die Verreibungstemperatur bei einer Temperatur von Umgebungstemperatur bis 110°C liegt, und zwar in Abhängigkeit davon, ob das für die Verreibung verwendete Milieu bei Umgebungstemperatur fest ist oder nicht.

13. Verfahren gemäß jedem der Ansprüche 4 bis 12,
dadurch **gekennzeichnet**, daß
die linearen oder verzweigten Monoalkohole aus Dodecanol und Oleylalkohol, die linearen oder verzweigten Polyole aus Propylenglycol oder Glycerin, der cycloaliphatische Alkohol aus Cyclohexanol, die aromatischen Alkohole aus Benzyl- oder Phenylethylalkohol, die Ester aus Isopropylmyristat und - palmitat, aus Triglyceriden von C₈₋₁₂-Fettsäuren, aus Triolein, Octylstearat, Benzoaten von Fettalkoholen, Pentaerythrittetracaprinat/caprat und aus Pentaerythrittetraisostearat und die Silicone aus in Form eines Öls vorliegenden Polyorganosiloxanen ausgewählt sind.

14. Verfahren gemäß Anspruch 13,
dadurch **gekennzeichnet**, daß
die Silicone Polyalkylsiloxane, Polyarylsiloxane, Polyalkylarylsiloxane, Gummi- und Harzprodukte von Silicon in Mischung mit flüchtigen Siliconen, mit oxethylierten oder oxpropylierten Gruppen modifizierte Polyorganosiloxane sind.

15. Dispersion, erhältlich gemäß dem in jedem der Ansprüche 4 bis 14 definierten Verfahren.

16. Verwendung der Dispersion gemäß jedem der Ansprüche 1 bis 3 und 15 zur Herstellung von Zusammensetzungen zum Schminken, von Sonnenmittel-Zusammensetzungen oder von Zusammensetzungen zur Färbung der Haare.

17. Kosmetische Zusammensetzung,
dadurch **gekennzeichnet**, daß
sie mindestens teilweise eine in jedem der Ansprüche 1 bis 3 oder 15 definierte Dispersion in einem kosmetisch geeigneten Milieu enthält.

18. Zusammensetzung gemäß Anspruch 17,
dadurch **gekennzeichnet**, daß
sie Melanin-Pigmente in Form von Partikeln, von denen mindestens 75% der Partikel eine Korngröße von weniger als 10 »m und vorzugsweise 85% eine Korngröße von weniger als 5 »m aufweisen, in Mengenanteilen von 0,001 bis 20 Gew.% enthält, bezogen auf das Gesamtgewicht der Zusammensetzung.

19. Zusammensetzung gemäß jedem der Ansprüche 17 und 18 zur Verwendung zum Schminken der Haut, der Augenbrauen und der Wimpern,
dadurch **gekennzeichnet**, daß
sie in fester oder pastenartiger, wasserfreier oder wässriger Form vorliegt.

20. Zusammensetzung gemäß jedem der Ansprüche 17 und 18 zum Schutz der menschlichen Epidermis vor der UV-Strahlung, dadurch **gekennzeichnet**, daß
sie in Form einer Suspension in Lösungsmitteln oder Fettkörpern, in Form von Emulsionen, Pomaden, Gelen, Feststoffstäbchen oder von Aerosol-Schäumen vorliegt.

21. Zusammensetzung gemäß jedem der Ansprüche 17 und 18, dadurch **gekennzeichnet**, daß
sie Fettkörper, organische Lösungsmittel, Silicone, Verdickungsmittel, Weichmacher, oberflächenaktive Mittel, Sonnenfiltermittel, Antischaummittel, hydratisierende Mittel, Parfüm-Produkte, Konservierungsstoffe, Antioxidantien, Beaufschlagungsmittel, Sequestriermittel, Behandlungsmittel, Treibmittel, alkalisch oder sauer stellende Mittel oder weitere Pigmente enthält.

22. Zusammensetzung gemäß Anspruch 20,
dadurch **gekennzeichnet**, daß
sie ein Nanopigment aus Metalloxid enthält, welches aus Oxiden von Titan, Zink, Cer oder Zirkon ausgewählt ist, wobei diese Nanopigmente einen mittleren Durchmesser von weniger als 100 nm aufweisen und umhüllt sind oder nicht.
